# EUROPEAN PATENT APPLICATION

(11) **EP 1 203 617 A1**
(43) Date of publication of application: **08.05.2002**
(21) Application number: 01127865.2
(22) Date of filing: 30.10.1995
(51) Int. Cl.: B01J 37/02, B01J 21/06

(54) **Titanium dioxide photo catalyst structure**

(30) Priority: 31.10.1994 JP 26747694
(62) Divisional of application: 95935582.7
(71) Applicant: KANAGAWA ACADEMY OF SCIENCE AND TECHNOLOGY, Kawasaki-shi, Kanagawa-ken 213 (JP); NIPPON SODA CO., LTD., Chiyoda-ku, Tokyo 100-8165 (JP)
(72) Inventor: Fukayama, Shingemichi, Odawara-shi Kanagawa-ken 250-02 (JP)
(74) Representative: Grünecker, Kinkeldey, Stockmair & Schwanhäusser Anwaltssozietät

(57) **Abstract**

The present invention relates to a titanium dioxide photocatalyst structure, a method for producing same and its use for removing carbon dioxide and air pollutants to render indoor space antibacterial.

## Description

### Technical Field

The present invention relates to a titanium dioxide photocatalyst structure that has excellent photocatalytic actions and light transmissivity and enables members of various substances, which require transparency particularly, to have photocatalytic actions. The present invention further relates to a method for producing such a titanium dioxide photocatalyst structure.

### Background Art

Heretofore, there have been known photocatalysts that exhibit activities, by which the decomposition and oxidation of substances are accelerated, when irradiated with light. Recently, attempts or the like have been made to remove air pollutants such as sulfur oxides and nitrogen oxides by utilizing the photocatalysts. Moreover, attempts have been further made to use titanium dioxides as the photocatalysts (see, for example, Japanese Patent Laid-Open Nos. 6-385/1994, 6-49677/1994 and 6-39285/1994 Official Gazettes).

By the way, in recent years, there has been a growing interest in globally environmental pollution. Meanwhile, the demand for removing substances such as CO₂, NOₓ and SOₓ has grown. Moreover, a plan for creating amenity space by eliminating toxic substances has been devised. Thus, the demands for deodorizing living space and for making the living space antibacterial, soil- resistant and mildew-proof have grown increasingly.

It is accordingly conceived that the aforementioned titanium-dioxide photocatalyst is utilized for removing such pollutants. However, in the case of the conventional titanium dioxide photocatalysts, generally, gaseous or liquid materials to be treated are introduced into a container accommodating the photocatalyst and are thus made to be in contact with the photocatalyst, and simultaneously, light is introduced from the exterior thereto and is applied onto the photocatalyst.

Further, in such a case, for the purpose of increasing the contact area between the material to be treated and the photocatalyst and efficiently applying the light onto the photocatalyst, attempts or the like have been made to produce the photocatalyst in minute- particle form or to hold the photocatalyst on a transparent base material.

However, in the case of the aforementioned conventional titanium dioxide photocatalyst, although the contact area between the photocatalyst and the material to be treated can be increased by, for instance, producing the photocatalyst in minute-particle form, the effective area of the photocatalyst, by which light is received, cannot be increased very much. Consequently, it is difficult to largely enhance the total catalysis effects thereof.

Further, in the case where the titanium dioxide photocatalyst is formed in film form on, for example, a glass substrate or the like, the titanium dioxide photocatalyst itself has low transparency. This is because it has been heretofore considered that methods suitable for forming a photocatalyst in film form to thereby obtain practical photocatalysis are limited to a method of forming a titanium dioxide sol on the substrate by sintering and a method of producing titanium dioxide in fine powder form, dissolving the powder by using a binder and then applying the dissolved powder onto the substrate. However, in the case of employing the former method, a photocatalyst, which has high activity and a certain measure of transparency, can be obtained, though it is necessary for obtaining the film, whose strength is sufficient for practical use, to set a sintering temperature at a value which is not lower than the softening temperature of glass. Thus, at least, it is impossible to form the photocatalyst on the glass substrate. Besides, regarding the light transmissivity, this photocatalyst tends to become clouded. It is difficult for this photocatalyst to transmit visible light to such an extent that the transparency can be obtained. In this sense, this photocatalyst is close to opaque. In contrast, in the case of the latter method, although the step of sintering is unnecessary, the photocatalyst becomes clouded and opaque because fine titanium dioxide powder is applied to the substrate.

Further, in the case of titanium dioxide produced in film form by performing a sol-gel method and CVD method which have been well known in the field of such a kind heretofore, the transparency can be ensured, whereas the activity of the catalyst, which has a practical level, is not obtained.

Thus, all of the conventional titanium dioxide photocatalysts, which exhibit the photocatalytic activities of practical levels, are substantially opaque.
Therefore, even in the case that this conventional photocatalyst is formed on, for example, the front surface of a transparent glass substrate or the like, light applied from the back surface of the glass substrate cannot effectively reach the front surface portion of the photocatalyst. Consequently, only light applied from the front surface portion, on which the photocatalyst is not formed, of the substrate can be utilized. Hence, in the case that the cleaning of indoor air is performed by forming this photocatalyst on, for instance, the surface of window pane, it naturally follows that the photocatalyst is formed on the surface of the glass, which faces the inside of a room. Thus, only light applied from the inside of the room that can be utilized for obtaining the photocatalytic activity.
Consequently, there has been a serious defect that sunlight cannot be utilized therefor.

Thus, in the case of the conventional titanium dioxide photocatalyst, titanium dioxide, which performs the photocatalysis, itself is substantially opaque.
Consequently, there occurs a limit to the enhancement of the photocatalytic activity. Moreover, the range of application of the photocatalyst is extremely limited.

The present invention is accomplished against the aforementioned background. The present invention aims at providing a titanium dioxide photocatalyst structure that has excellent photocatalytic actions and light transmissivity and enables members of various substances,
which require transparency particularly, to have photocatalytic actions and further aims at providing a method for producing such a photocatalyst structure.

### Disclosure of Invention

To solve the aforesaid problem, in accordance with the present invention, there is provided a titanium dioxide photocatalyst structure (Structure 1) comprising:
a titanium dioxide film having at least photocatalytic activity and light transmittance, which is not less than 50 % for light having a wavelength of 550 nm, is formed on a transparent substrate.

As an embodiment of Structure 1, there is provided a photocatalyst structure (Structure 2), wherein the titanium dioxide film is 0.1 to 5 µm in thickness.

As an embodiment of Structure 1 or Structure 2, there is provided a photocatalyst structure (Structure 3), wherein the titanium dioxide film contains at least anatasecrystals.

As an embodiment of Structure 1, Structure 2 or Structure 3, there is provided a photocatalyst structure (Structure 4), wherein a precoat film having transparency is disposed between the transparent substrate and the titanium dioxide film.

As an embodiment of Structure 4, there is provided a photocatalyst structure (Structure 5), wherein the precoat film is 0.02 to 0.2 µm in thickness.

As an embodiment of Structure 1, Structure 2, Structure 3, Structure 4 or Structure 5, there is provided a photocatalyst structure (Structure 6), wherein the transparent substrate is made of glass.

As an embodiment of Structure 1, Structure 2, Structure 3, Structure 4, Structure 5 or Structure 6, there is provided a photocatalyst structure (Structure 7), wherein the precoat film is made mainly of SiO₂.

Further, in accordance with the present invention, there is provided a method (Structure 8) for producing a titanium dioxide photocatalyst structure which is one of Structure 1 to Structure 7. In the case of this method, the titanium dioxide photocatalyst structure is produced by performing a producing process which includes the step of forming a titanium dioxide film on a transparent substrate by performing what is called a pyro-sol method, a dipping method, a printing method or a CVD method.

In accordance with the foregoing Structure 1, a titanium dioxide film, which has at least photocatalytic activity and light transmittance corresponding to light having a wavelength of 550 nm is not less than 50 %, is formed on a transparent substrate. Thereby, the titanium dioxide photocatalyst structure can have excellent photocatalytic action and optical transmissivity. Moreover, the titanium dioxide photocatalyst structures can be used as members composing various structures such as a glass window, which are especially required to have light transmissivity.

This is owing to the fact that as a result of setting the titanium dioxide film in such a manner that the light transmittance corresponding to light having the wavelength of 550 nm is not less than 50 %, the substantially increasing of light irradiation efficiency required to obtain photocatalytic activities can be easily achieved and simultaneously, the transparency corresponding to visible light can be ensured. Namely, if setting the titanium dioxide film in such a manner that the light transmittance corresponding to light having the wavelength of 550 nm is not less than 50 %, the titanium dioxide film inevitably has the transmissivity corresponding to light, which gives the photocatalytic activity (and which has the wavelength of about 400 nm), in such a way that the degree of the transmissivity is sufficient to effectively utilize light applied thereto from the front and back thereof. Therefore, when both of the front and back surfaces of this titanium dioxide photocatalyst structure are irradiated with different light, respectively, the light rays respectively coming from both of the surfaces thereof reach the surface portion, which is in contact with the exterior, of the titanium dioxide film in such a manner as to be added to each other. Namely, the efficiency in applying light onto the surface porion of the titanium dioxide film can be substantially increased. Thereby, the photocatalytic activity of the surface portion of the titanium dioxide film can be substantially increased in response to this, so that excellent photocatalytic activity can be obtained. Simultaneously, as a consequence of setting the titanium dioxide film in such a manner that the light transmittance corresponding to light having the wavelength of 550 nm is not less than 50 %, the sufficient transparency corresponding to visible light can be secured inevitably. Consequently, this titanium dioxide photocatalyst structure can be used as a member of various structures especially required to have the transparency, for example, a glass window. The present invention can have distinguished advantages in that actions of eliminating carbon dioxide and air pollutants (for example, NOₓ and SOₓ) from indoor space, of deodorizing the indoor space and of making the indoor space antibacterial, soil-resistant and mildew-proof are achieved by the window pane itself without using special equipment. Additionally, the present invention can obtain eminent merits in that in the case of cleaning the room by applying the photocatalyst structure to the window pane, sunlight can be extremely utilized. Moreover, especially, in the case of applying the photocatalyst structure of the present invention to a building or the like, in which glass materials are highly used, of the type that has become common in recent years, the photocatalyst structure of the present invention has immeasurable advantages in cleaning the living space. In addition, the photocatalyst structure of the present invention can be applied to a glass door or the like of a shelf, which includes the door or the like, for storing, for instance, precision devices such as a camera which should be kept away from molds and corrosion. Thus, the range of application of the photocatalyst structure of the present invention is extremely wide.

In accordance with the foregoing Structure 2, a titanium dioxide film having sufficient photocatalytic activity and simultaneously having the light transmittance, which is not less than 50 % correspondingly to light having a wavelength of 550 nm, can be obtained by setting the thickness of the titanium dioxide tin film at a value of 1 to 5 µm. In the case that the thickness of the photocatalyst structure is less than 0.1 µm, sufficient photocatalytic activity cannot be obtained. In contrast, in the case that the thickness of the photocatalyst structure exceeds 5 µm, the light transmittance corresponding to the light having the wavelength of 550 nm is less than 50 %. Consequently, sufficient transparency cannot be obtained.

In accordance with Structure 3, the titanium dioxide film contains anatase crystals. Thereby, the photocatalyst structure can further excels in photocatalytic activity.

In accordance with Structure 4, a precoat film having transparency is disposed between the transparent substrate and the titanium dioxide film. Thus, the material of the transparent substrate penetrates into the titanium dioxide film, so that the photocatalytic activity of the titanium film can be prevented from being degraded. Moreover, the range of materials of the transparent substrate to choose can be extended. Furthermore, in the case of forming a titanium dioxide film directly on the transparent substrate, the titanium dioxide film should have a thickness sufficient to the extent that even when the material of the transparent substrate penetrates into the titanium dioxide film, the material cannot reach titanium dioxide on which charge separation action should be exerted. The present invention, however, eliminates the necessity of making the film thick to such an extent. Thus, even when the titanium dioxide film is made to be extremely thin regardless of what kind of materials the substrate employs, the photocatalytic activity can be sufficiently enhanced.

In the case that the thickness of the precoat film is 0.02 to 0.2 µm similarly as in the case of Structure 5, even when taking materials, which can be employed as those of the precoat film, into consideration, the photocatalyst structure of the present invention can obtain advantages in that sufficient transparency can be ensured and that the penetration of the material of the substrate can be blocked. Conversely, in the case that the thickness of the precoat film is less than 0.02 µm, it is difficult to have a sufficient effect on the blockage of the penetration of the material. Further, even in the case that the film, whose thickness exceeds 2 µm, is formed, the photocatalyst structure cannot have further advantageous effects on the blockage of the penetration of the material. Moreover, an operation of forming the film becomes complicated. Furthermore, if the film is made of some material, the sufficient transparency cannot be ensured.

In the case that glass is used as the transparent substrate similarly as in the case of Structure 6, the extremely wide range of application of the photocatalyst structure of the present invention can be achieved, as previously described. In this case, if the precoat film is made of SiO₂ similarly as in the case of Structure 7, the best transparency and the highest effects on the blockage of can be secured.

Furthermore, in accordance with Structure 8, the titanium dioxide photocatalyst structure according to one of Structure 1 to Structure 7 can be relatively easily obtained.

### Brief Description of Drawings

FIG. 1 is a partially sectional diagram for illustrating the configuration of a titanium dioxide photocatalyst structure according to Example 1;
FIG. 2 is a partially sectional diagram for illustrating the configuration of a titanium dioxide photocatalyst structure according to Example 7;
FIG. 3 is a diagram for showing a table in which the thickness of films of Examples according to the present invention and Comparative Examples, results of measurement of photocatalytic activities thereof and results of measurement of the light transmittance thereof are presented;
FIG. 4 is a diagram for showing a graph which represents results of measurement of fat splitting activities of Example 16 to Example 18 according to the present invention; and
FIG. 5 is a diagram for showing a graph which represents results of measurement of fat splitting activities of Example 19 and Example 20 according to the present invention.

### Best Mode for Carrying Out the Invention

### (Example 1)

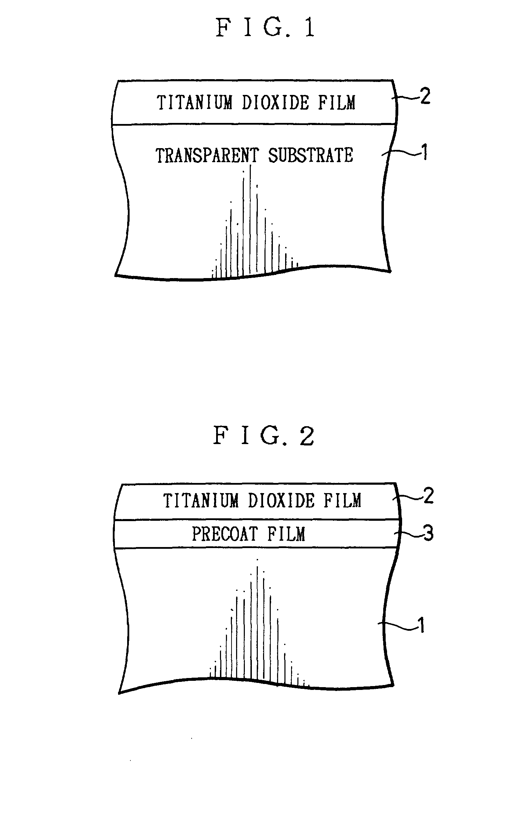

FIG. 1 is a partially sectional diagram for illustrating the configuration of a titanium dioxide photocatalyst structure according to Example 1 of the photocatalyst structure of the present invention. Hereinafter, Example 1 of the titanium dioxide photocatalyst structure and a method for producing this titanium dioxide photocatalyst structure will be described with reference to FIG. 1.

As shown in FIG. 1, in the case of this example of the titanium dioxide photocatalyst structure, a titanium dioxide film 2 is formed on a transparent substrate 1.

The transparent substrate 1 is a soda lime glass substrate whose thickness, longitudinal size and lateral size are 1, 100 and 50 mm, respectively.

The film 2 is a titanium dioxide film which contains anatase crystals and is 4.8 µm in thickness.

The aforementioned titanium dioxide photocatalyst structure was produced by performing the following process.

First, a transparent substrate 1 was produced by extracting a soda lime glass whose thickness, longitudinal size and lateral size were 1 mm, 100 mm and 50 mm, respectively.

Next, a raw-material solution, in which the concentration of titanium isopropoxide was adjusted to 0.5 mol/L, was made by dissolving titanium isopropoxide in acetylacetone solvent as the material of a titanium dioxide film.

Subsequently, the transparent substrate 1 was set in a pyro-sol film forming device. Further, the transparent substrate 1 was preliminarily heated to 500 degrees centigrade. Moreover, the raw-material solution underwent ultrasonic atomization and was then introduced to the surface of the transparent substrate 1 at a rate of 20 ml/min. Then, an operation of forming a film was performed in the period of about 60 minutes. Consequently, there had been obtained a titanium dioxide photocatalyst structure in which this titanium dioxide 2, whose thickness was 4.8 µm, was formed on the transparent substrate 1. Incidentally, according to a result of analysis of this titanium dioxide film 2 based on X-ray diffraction, it was verified that this film 2 contained anatase crystals.

Next, the photocatalytic activity and light transmittance of the obtained titanium dioxide photocatalyst structure were measured by performing the following method.

### Method for Measuring Photocatalytic Activity

The titanium dioxide photocatalyst structure was placed in the bottom of a cylindrical glass gastight enclosure, whose capacity was 1.5 L, in such a way that the titanium dioxide film 2 was the upper part thereof. Then, acetaldehyde was introduced into this enclosure so that the concentration of acetaldehyde was 1300 ppm. Next, the titanium dioxide photocatalyst structure was irradiated with light from above the surface of the titanium dioxide film 2 by using three 10-W black lights. At that time, the illuminance measured on the surface of the titanium dioxide film 2 was 1.2 mW/cm2. Thereafter, the quantitative analysis of acetaldehyde contained in the glass gastight enclosure was performed by using a gas chromatograph with FID. Thus, the decrement of the amount of acetaldehyde, which was caused after the photocatalyst structure was irradiated with light, was obtained. Further, the obtained decrement of the amount of acetaldehyde was defined as the degree of the photocatalytic activity.

### Method for Measuring Light Transmittance

Titanium dioxide photocatalyst structure of Example 1 was first set in a device for measuring light transmittance (namely, UV-3100PC manufactured by Shimadzu Corporation). Then, the light transmittance corresponding to light, whose wavelength is 550 nm, was measured.

According to a result of the measurement by the herein-aforementioned method, what is called the decomposition activity was 10.5 µl/min and the light transmittance was 70 %. Thus, it was verified that this titanium dioxide photocatalyst structure had excellent photocatalytic activity and sufficient transparency.

Then, the titanium dioxide photocatalyst structure was again placed in the bottom of the aforesaid cylindrical glass gastight enclosure by being turned upside down, namely, in such a way that the titanium dioxide film 2 faced the bottom of the enclosure. Subsequently, the photocatalytic activity of the photocatalyst structure was measured by irradiating the photocatalyst with light coming from the similar direction, namely, from the side, on which the titanium dioxide film 2 was not formed, of the transparent substrate 1. At that time, it was verified that the photocatalytic activity, whose value was nearly close to that obtained in the aforementioned case, could be obtained. This result shows that light applied from the back surface of the transparent substrate 1 contributes to the photocatalytic activity of the titanium dioxide film 2 as well as the light applied from the front surface of the transparent substrate 1 and that the photocatalytic effects thereof can be considerably enhanced by applying light to both of the sides of the photocatalyst structure.

### (Example 2 to Example 6)

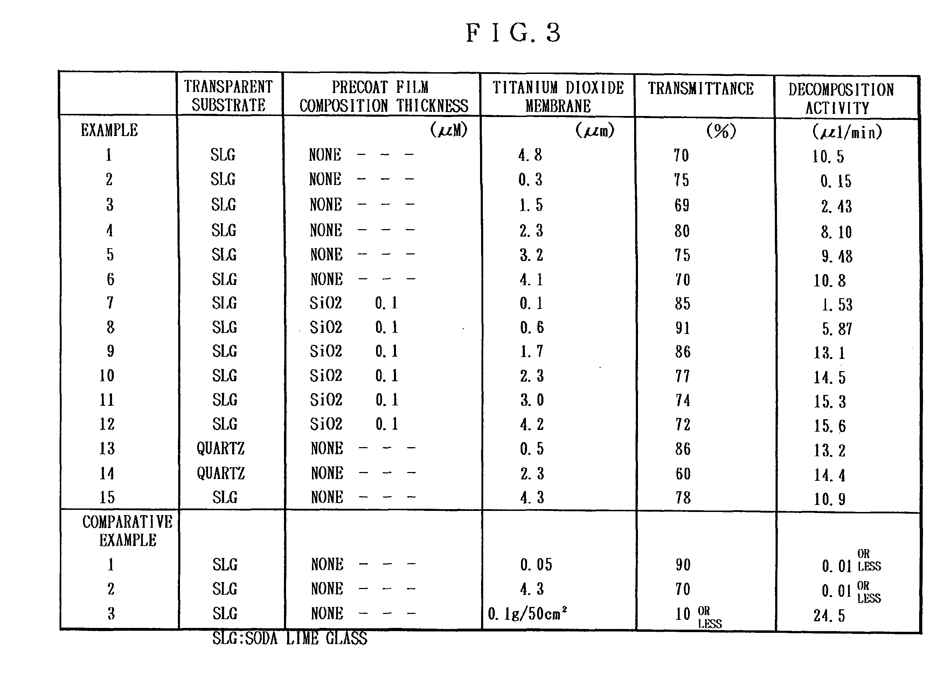

These Examples have structures, which are similar to the structure of Example 1 except that the thickness of the titanium dioxide film 2 is different from that of the film 2 of Example 1, and are produced by a producing method similar to that employed in the case of Example 1. Data representing the thickness of each Example and results of measurement of the photocatalytic activity and light transmittance are shown in FIG. 3 in tabular form. Further, the detailed description of the data is omitted.

As is seen from the table of FIG. 3, each of these Examples has excellent photocatalytic activity and sufficient transparency.

### (Example 7 to Example 12)

These Examples have structures, which are similar to the structure of Example 1 except that a precoat film 3 constituted by a SiO2 film is formed between the titanium dioxide film 2 and the transparent substrate 1 by a dip coating as illustrated in FIG. 2, and are produced by a producing method similar to that employed in the case of Example 1. Data representing the thickness of each Example and results of measurement of the photocatalytic activity and light transmittance are shown in FIG. 3 in tabular form. Further, the detailed description of the data is omitted.

As is seen from the table of FIG. 3, even when the thickness of the titanium dioxide film 2 is reduced, excellent photocatalytic activities are exhibited, in comparison with Example 1 to Example 6 which do not have the precoat film 3. Thus, further higher transparency can be ensured.

### (Example 13 to Example 14)

These Examples have structures, which are similar to the structure of Example 1 except that the soda lime glass of Example 1 is replaced with quartz glass, and are produced by a producing method similar to that employed in the case of Example 1. Data representing the thickness of each Example and results of measurement of the photocatalytic activity and light transmittance areshown in FIG. 3 in tabular form. Further, the detailed description of the data is omitted.

As is seen from the table of FIG. 3, even though the Examples do not have the precoat film 3, excellent photocatalytic activities are exhibited, because quartz glass is used as the material of the transparent substrate 1. In addition, these Examples have sufficient transparency.

### (Example 15)

This Example has a structure, which is similar to the structure of Example 1 except that the temperature at the time of forming the titanium dioxide film is changed into 380 degrees centigrade and that the step of performing heat treatment in the period of 60 minutes at the temperature of 400 degrees centigrade under airatmosphere is added, and are produced by a producing method similar to that employed in the case of Example 1. Data representing the thickness of each Example and results of measurement of the photocatalytic activity and light transmittance are shown in FIG. 3 in tabular form. Further, the detailed description of the data is omitted.

As is seen from the table of FIG. 3, each of these Examples has excellent photocatalytic activity and sufficient transparency.

### (Example 16)

In the case of this Example, the precoat film 3 constituted by a SiO₂ film having a thickness of 0.06 µm was formed on the transparent substrate 1. Moreover, the titanium dioxide film 2 having a thickness of 0.8 µm was formed by what is called a dipping method.

A soda lime glass plate, whose thickness, longitudinal size and lateral size are 1 mm, 100 mm and 50 mm, respectively, was used as the transparent substrate 1. This transparent substrate 1 was slowly dipped into a vessel, which contains silicon alkoxide solution (incidentally, the trade name thereof was "ATOLON NSi-500" and was manufactured by Nippon Soda Co., Ltd.) of 800 ml and has a width of 100 mm, a depth of 50 mm and a height of 200 mm, respectively. Thereafter, this transparent substrate 1 was pulled up therefrom at a speed of 10 cm/min. This substrate was then dried at the temperature of 150 degrees centigrade. Subsequently, the substrate was sintered at the temperature of 500 degrees centigrade in the period of 1 hour. Thus, a SiO2 film, whose thickness was 0.06 µm, was formed on the transparent substrate 1 as the precoat film 3.

Next, the glass substrate provided with the precoat film was slowly dipped into 800 ml of an organic titanium solution for dipping (incidentally, the trade name thereof was "ATOLON NTi-500" and was manufactured by Nippon Soda Co., Ltd.), which was obtained by causing titanium tetraisoproxide to react with organic solvent. Thereafter, the substrate was slowly pulled up therefromat a speed of 10 cm/min. This substrate was then driedat the temperature of 120 degrees centigrade. Subsequently, the substrate was sintered at thetemperature of 500 degrees centigrade in the period of 1hour. Thus, a titanium dioxide film was formed on the precoat film. This process for forming a titanium dioxide film, which had the steps of dipping the substrate into chemicals and then drying and sintering the substrate, was repeated 10 times. Thus, the thickness of the titanium dioxide became 0.8 µm, so that the titanium dioxide photocatalyst structure of this Example was obtained.

The titanium dioxide photocatalyst structure obtained in this way has the light transmittance of 81 % correspondingly to light having the wavelength of 550 nm. Further, regarding this Example, a fat splitting activity was taken up as one of the photocatalytic activities and was evaluated as follows.

### Evaluation of Fat Splitting Activity

The aforementioned titanium dioxide photocatalyst structure was extracted as a 50-by-50-mm test piece. Then, the entire surface of the test piece was softly wiped with tissue paper which had soaked commercially available salad oil. Thus, an amount of applied salad oil was adjusted to 0.1 mg/cm2 by applying the salad oil thereto and wiping the salad oil therefrom. The initial amount of salad oil was measured by weighing the glass plate by using a precision balance whose weight accuracy is 0.1 mg. This substrate, to which the salad oil had been applied, was then irradiated with ultraviolet rays coming from ultraviolet lamps (namely, three 10-W black lights FL10BLB manufactured by Matsushita Electric Works,Ltd., were arranged in a low and were used for applying the ultraviolet rays) for a predetermined time period, by regulating the distance between each ultraviolet lamp and the surface of the test piece in such a manner that the ultraviolet intensity measured by the ultraviolet-intensity detector (manufactured by Ultraviolet Corporation) was 3 mW/cm2. After the predetermined time period has passed, the amount of the split salad oil (fat) was found by measuring the weight of the glass substrate.

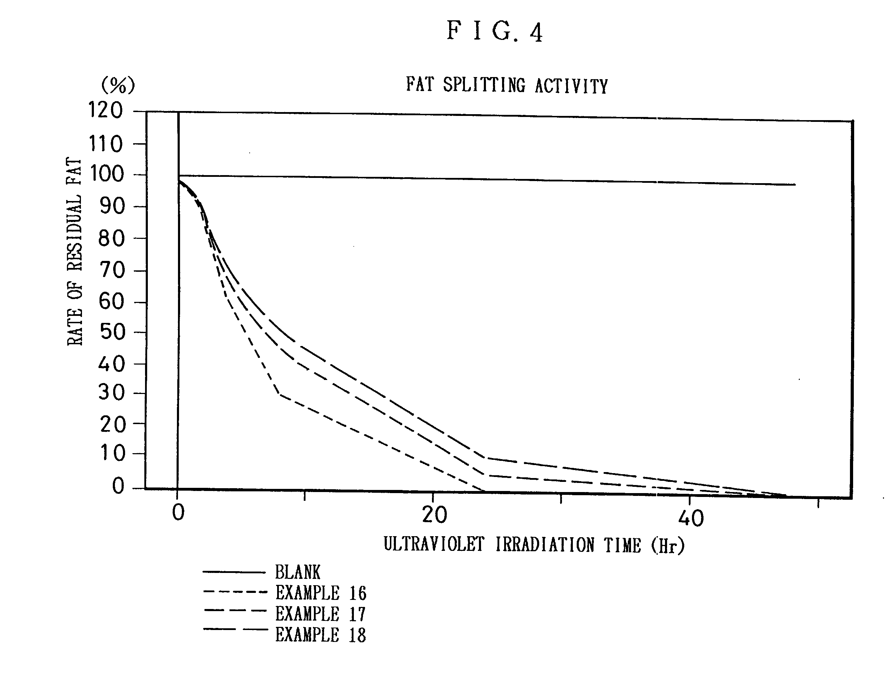

FIG. 4 illustrates a graph which shows the fat splitting characteristics of the photocatalyst structure of Example 16. Incidentally, in the graph of FIG. 4, the vertical axis represents the rate of residual fat (in %); and the horizontal axis a time period (in hours) in which ultraviolet was applied. Further, similarly as in the herein-aforementioned case, salad oil was applied to the glass substrate provided only with a precoat film, on which no titanium dioxide film was formed. Then, ultraviolet was applied thereto (from the black light). After the predetermined time period passed, a change in weight of the glass substrate was measured. Thus, the resultant data was obtained as data in the case of a "blank" test piece. The result in this case is also shown in the graph of FIG. 4. As is obvious from FIG. 4, this titanium dioxide photocatalyst structure has excellent fat splitting activity.

### (Example 17)

In the case of this Example, the precoat film 3 constituted by a SiO2 film having a thickness of 0.06 µm was formed on the transparent substrate 1.
Further, a titanium dioxide film having a thickness of 0.6 µ m was formed thereon by performing what was called a spraying method.

Similarly as in the case of Example 16, the SiO2 film having a thickness of 0.06 µm was formed as the precoat film 3 by using a soda lime plate whose thickness, longitudinal size and lateral size are 1 mm, 100 mm and 50 mm, respectively.

Next, the transparent substrate 1, on which this precoat film 3 was formed, was leaned against the inner part of a box-type heater which had been heated to 500 degrees centigrade. Then, 10 shots of an organic solvent solution of titanium acetylacetonato obtained by causing a reaction between 140 g of titanium tetraisopropoxide and 200 g of acetylacetone were sprayed onto the precoat film 3 of the transparent substrate1. Subsequently, the titanium dioxide film 2 having a thickness of 0.6 µm was formed by performing thermal decomposition thereon.

The light transmittance of the titanium dioxide photocatalyst structure corresponding to light having the wavelength of 550 nm was 78 %. Further, the fat splitting activity acting as the photocatalytic activity was measured similarly as in the case of Example 16. A result of this measurement is shown in FIG. 4 together with a result of the measurement in the case of Example 16.

### (Example 18)

In the case of this Example, the precoat film 3 constituted by a SiO2 film having a thickness of 0.06 µm was formed on the transparent substrate 1.
Further, a titanium dioxide film having a thickness of 0.4 µ m was formed thereon by performing what was called a printing method.

Similarly as in the case of Example 16, the SiO₂ film having a thickness of 0.06 µm was formed as the precoat film 3 by using a soda lime plate whose thickness, longitudinal size and lateral size are 1 mm, 100 mm and 50 mm, respectively.

Next, a screen process printing was performed by a printing machine, which was provided with a 400-mesh screen, on the aforementioned precoat film 3 by using a solvent obtained by causing a reaction and dissolution among 70 g of titanium tetraisopropoxide, 100 g of ethyl cellulose and 1200 g of organic solvent. Upon completion of printing, the film was put into a stationary state in the period of 5 min. Then, the leveling of the film was performed. Thereafter, the film was sintered in an electric furnace which had been heated to 500 degrees centigrade. This printing and sintering process was repeated 5 times. Thus, the thickness of the titanium dioxide became 0.4 µm, so that the titanium dioxide photocatalyst structure of this Example was obtained.

The light transmittance of this photocatalyst structure corresponding to the wavelength of 550 nm was 86 %. Further, similarly as in the case of Example 16, the fat splitting activity was measured. Thus, a result of this measurement was obtained as shown in the graph of FIG. 4.

### (Example 19)

In the case of this Example, the precoat film 3 constituted by a SiO₂ film having a thickness of 0.04 µm was formed on the transparent substrate 1 by performing the pyro-sol method. Moreover, the titanium dioxide film 2 having a thickness of 0.4 µm was formed thereon by the dipping method.

The transparent substrate 1 constituted by a soda lime glass plate, whose thickness, longitudinal size and lateral size are 1 mm, 100 mm and 50 mm, respectively, was set in the pyro-sol film forming device. Subsequently, organic silicon solution (incidentally, the trade name thereof was "ATOLON NSi-500" and was manufactured by Nippon Soda Co., Ltd.) underwent ultrasonic atomization and was then introduced to the glass substrate, which had been heated to 500 degrees centigrade, at a rate of 20 ml/min in the time period of 2 min. Thus, the silicon-dioxide precoat film 3, whose thickness was 0.04 µm, was formed.

Next, similarly as in the case of Example 16, a titanium dioxide film was formed on this precoat film 3 by performing the dipping method. This process for forming a titanium dioxide film, which had the steps of dipping the substrate into chemicals and then soaking, drying and sintering the substrate, was repeated 5 times. Thereby, the thickness of the titanium dioxide became 0.4 µ m, so that the titanium dioxide photocatalyst structure, in which the transparent titanium dioxide film 2 was formed, was obtained.

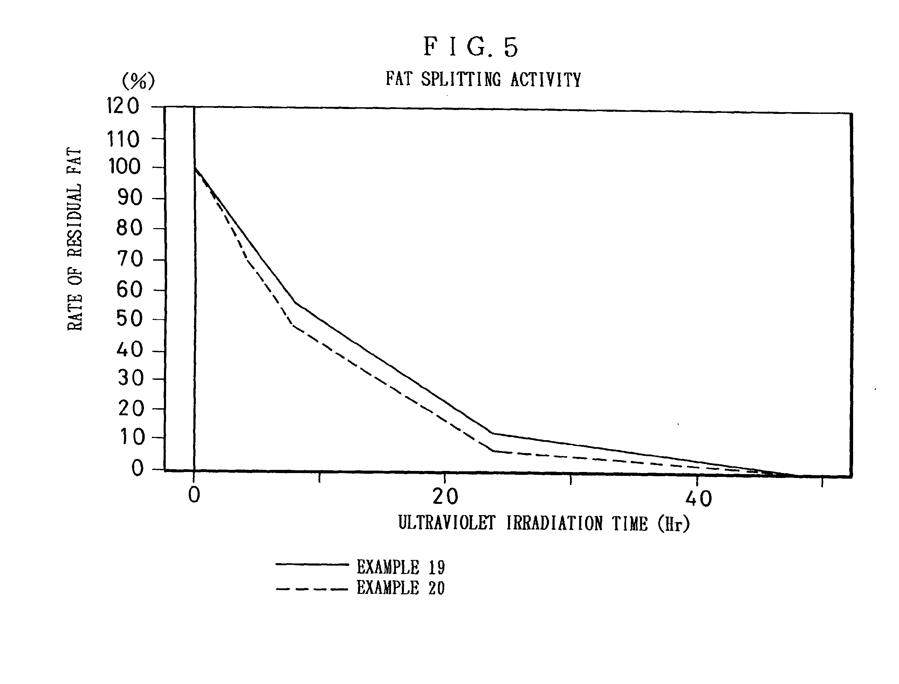

The light transmittance of this photocatalyst structure corresponding to the wavelength of 550 nm was 89 %. Further, similarly as in the case of Example 16, the fat splitting activity was measured. Thus, a result of this measurement was obtained as shown in the graph of FIG. 5.

### (Example 20)

In the case of this Example, the precoat film 3 constituted by a SiO₂ film having a thickness of 0.06 µm was formed on the transparent substrate 1. Moreover, the titanium dioxide film 2 having a thickness of 0.6 µm was formed thereon by what is called a CVD method.

The transparent substrate 1 constituted by a soda lime glass plate, whose thickness, longitudinal size and lateral size are 1 mm, 100 mm and 50 mm, respectively, was used as the transparent substrate 1. Moreover, the precoat film 3, which was made of silicon dioxide and was 0.06 µm in thickness, was formed by performing the same method as used in the case of Example 16.

Next, the transparent substrate 1, on which this precoat film 3 was formed, was set in an atmospheric pressure CVD film forming device. Further, titanium tetraisopropoxide was prepared in a carbureter heated to 200 degrees centigrade. Then, gaseous nitrogen was introduced into the carbureter at the flow rate of 50ml/min to thereby cause the bubbling of titanium tetraisopropoxide. Subsequently, the vapor of titanium alkoxide was introduced through a heated conduit into a film forming portion in which the glass substrate was set. The film forming portion was heated to 500 degrees centigrade. Furthermore, air was also introduced thereinto at the rate of 200 ml/min. Thus, an operation of forming a film was performed in the period of 5 minutes. Thereby, the titanium dioxide film 2 having a thickness of 0.6 µm was formed on the precoat film 3, so that the titanium dioxide photocatalyst structure was obtained.

The light transmittance of this photocatalyst structure corresponding to the wavelength of 550 nm was 84 %. Further, similarly as in the case of Example 16, the fat splitting activity was measured. Thus, a result of this measurement was obtained as shown in the graph of FIG. 5.

### (Comparative Example 1)

This Comparative Example has a structure, which is similar to the structure of Example 1 except that the thickness of the titanium dioxide film 2 is small, namely, 0.05 µm, and was produced by a producing method similar to that employed in the case of Example 1. Data representing the thickness of this Comparative Example and results of measurement of the photocatalytic activity and light transmittance are shown in FIG. 3 in tabular form. Further, the detailed description of the data is omitted.

As is seen from the table of FIG. 3, this Comparative Example has good transparency but exhibits little photocatalytic activity.

### (Comparative Example 2)

This Example has a structure, which is similar to the structure of Example 1 except that the temperature at the time of forming the titanium dioxide film was changed into 380 degrees centigrade, and was produced by a producing method similar to that employed in the case of Example 1. Data representing the thickness of this Comparative Example and results of measurement of the photocatalytic activity and light transmittance are shown in FIG. 3 in tabular form. Further, the detailed description of the data is omitted. Incidentally, according to a result of analysis of the titanium dioxide film 2, which was produced in this way, based on X- ray diffraction, it was verified that this film 2 did not contained anatase crystals at all.

As is seen from the table of FIG. 3, thisComparative Example has sufficient transparency but exhibits little photocatalytic activity.

### (Comparative Example 3)

This Example has a structure, which is similar to the structure of Example 1 except that the titanium dioxide film 2 was formed by using a method of applying 0.1 g of a solution, which was obtained by dispersing titanium dioxide powder (namely, "P-25" manufactured by NIPPON AEROSIL CORPORATION) into water, to the substrate, instead of the pyro-sol method, and was produced by a producing method similar to that employed in the case of Example 1. Data representing the thickness of this Comparative Example and results of measurement of the photocatalytic activity and light transmittance are shown in FIG. 3 in tabular form. Further, the detailed description of the data is omitted.

As is seen from the table of FIG. 3, this Comparative Example has high photocatalytic activity but has little transparency.

Incidentally, there is no particular restrain on the material of the transparent substrate used in the photocatalyst structure of the present invention, as long as the transparent substrate has optically predetermined transparency. To adduce actual examples, pyrex glass, quartz glass, lead glass and soda lime glass and so forth may be employed. Practically, inexpensive soda lime glass is mainly used. Further, quartz glass, which contains no alkaline ingredients such as sodium, and borosilicate glass, which contains little alkaline ingredients such as sodium, may be preferably used in the glass substrate. In the case where the transparent substrate is made of soda lime glass, the photocatalytic action of the titanium dioxide film is hindered by alkaline ingredients such as sodium, which are diffused from the substrate. It is, therefore, preferable for preventing the diffusion to provide a precoat film on the transparent substrate. In this case, the photocatalyst structure can be advantageously used even when the transparent substrate, in which alkaline ingredients such as inexpensive soda lime glass, may be diffused.

The titanium dioxide film has a thickness of 0.1 to 5 µm. If not more than 0.1 µm, the film exhibits the transparency but has low photocatalytic activity. Thus, the photocatalyst structure loses the practicality. In contrast, if exceeds 5 µm, the photocatalyst structure has advantages in that high photocatalytic activity can be maintained and that the risk of coloration due to optical interference can be reduced. The photocatalyst structure, however, tends to have defects in that the film becomes clouded and inclined and that it takes much time to peel and form a film.

Further, it is possible to utilize a titanium dioxide, which is provided in the vicinity of the surface of the film, as a photocatalyst structure by setting the thickness of the titanium dioxide film, which should be formed, at a large value, for example, 0.3 µm to 5.0 µm and by setting the concentration of sodium, which is contained in the titanium dioxide film, in such a manner as to change decreasingly in the direction of thickness. In this case, the precoat film can be omitted.

The precoat film has a thickness of 0.02 to 0.2 µm. If the thickness thereof is not more than 0.02 µm,the ability to prevent the diffusion of alkaline ingredients is reduced. In contrast, if the thickness thereof is not less than 0.2 µ m, there is no hindrance to the ability to prevent the diffusion of alkaline ingredients. However, the light transmissivity is reduced and an operation of forming a film becomes complex. Because the diffusion of alkaline ingredients such as sodium from the substrate is prevented by the precoat film, the thickness of the titanium dioxide film can be reduced. Moreover, the titanium dioxide film, whose transparency is further higher in the visible range, can be formed.

As long as the light transmittance in the visible range is high and the diffusion of sodium from the substrate can be prevented, there is no restrain on the composition of a precoat film. For instance, a silicone dioxide film, a tin oxide film, an indium-doped tin oxide film, an indium oxide film, a tin-doped indium oxide film, a germanium oxide film, an aluminum oxide film, zirconium oxide film and a SiO₂+MOₓ film (incidentally, MOₓ represents a kind of metallic oxide selected from the group of P₂O₅, B₂O₃, ZrO₂, TiO₂ and Ta₂O₅) can be cited as examples of the precoat film. From the view of the ability to prevent the diffusion of alkaline ingredients, a silicone dioxide film or a film, in which 5 % by weight of P₂O₅ is added to SiO₂, is particularly preferable

Moreover, the condition necessary to obtain a titanium dioxide film, which has a high photocatalytic activity, is that this film contains anatase crystals. When the temperature, at which the film is formed or at which the heat treatment is performed after forming the film, is high, the anatase crystals causes phase transition. As a result, a part of the anatase crystals are changed into rutile crystals. Therefore, an anatase-type titanium dioxide film containing rutile crystals is preferably used. It is, however, undesired that all of the anatase crystals are changed into rutile crystals at a high temperature. This is because of the fact that in such a case, owing to the phase transition, the titanium dioxide becomes clouded and thus the light transmittance in the visible range is decreased.

In the case of the photocatalyst structure of the present invention, although the pyro-sol method is the most suitable for forming a film, commonly known methods for forming films can be employed for forming both of the precoat film and the titanium dioxide film. Namely, a sputtering method, an electron beam evaporation method, an ion plating method, a chemical vapor deposition (CVD) method, a spraying method, a dipping method and so forth may be applied to the photocatalyst structure of the present invention by devising a process for controlling the formation of a film. Incidentally, methods, such as the pyro-sol method and the spraying method, for spraying a mist at normal pressure are preferable, because the application of such methods to the case of actually and industrially manufacturing photocatalyst structures can be achieved by spraying a mist during a glass plate is still hot in the process of cooling the glass plate which is being produced. Additionally, film forming methods, in which a substrate should be maintained at a high temperature, which is not lower than the softening temperature of glass, for example, at a high temperature, which is not lower than 600 degrees centigrade, are undesired because such methods cause the deformation of the substrate and accelerate the diffusion of alkaline ingredients.

What is called the pyro-sol method is desired as the method for forming films, for the following reasons. First, inexpensive titanium alkoxide of high purity is used as the material. Further, the films can be formed at a high speed. Moreover, a high-activity titanium dioxide film containing anatase crystals can be obtained in such a way as to be highly uniform and have a large area. Furthermore, the formation of the films can be achieved at a temperature which is not higher than the softening temperature of glass, namely, at a temperature which is 400 to 500 degrees centigrade or so. Such a temperature is an adequate temperature at which the diffusion of sodium can be retarded. Further, the diffusion thereof can be blocked by a precoat layer. The pyro-sol method is a kind of an atmospheric pressure chemical vapor cracking method (a CVD method) and is to form the precoat film or the titanium dioxide by carrying out the process of performing the vapor-phase transport of a mist, which has undergone the ultrasonic atomization, to the substrate, which has been heated to 400 to 550 degrees centigrade, and further thermally decomposing the mist on the substrate.

Chemicals for producing precoat films are as follows. Namely, chemicals for producing SiO₂ are silicon alkoxides, such as Si(OCH₃)₄, Si(OC₂H₅)₄ and SiCH₃(OCH₃)₃, and condensation products thereof and silicon halide. Further, chemicals for producing tin oxide are Sn(OCH₃)₄,

Sn(OC₂H₅)₄, Sn(OC₄H₉)₄, Sn(AcAc)₄, Sn(OCOC₄H₁₅)₄, Sn C₁₄ and so on. Moreover, chemicals for producing indium oxide are In(OCH₃)₃, In(OC₂H₅)₃, InCl₃, In(AcAc)₃, In(NO)₃nH₂₀ and so forth. Furthermore, chemicals for producing germanium oxide are Ge(OC₂H₅)₄, Ge(OC₄H₉)₄, GeCl₄ and so forth. Further, chemicals for producing aluminum oxide are Al(OC₂H₅)₃, Al(OiC₃H₇)₃, Al(OC₄H₉)₃, Al(AcAc)₃, Al(NO₃)₃₉H₂₀ and so on. Moreover, chemicals for producing phosphorus pentaoxide are P(OC₂H₅)₃, PO(OCH₃)₃, PO(OC₂H₅)₃, H₃PO₄, P₂O₅ and so forth. Additionally, chemicals for producing boron oxide are B(OCH₃)₃, B(OC₂H₅)₃, B(OC₄H₉)₃, B(AcAc)₃, BCl₁₃, H₃BO₃ and so on. Among these, usually available chemicals are used. Incidentally, in the chemical formula, "AcAc" designates C₅H₇O₂ (namely, acetylacetonato).

Manufacturing chemicals for producing titanium dioxide films are as follows: titanium alkoxides such as Ti(OC₂H₅)₄, Ti(OiC₃H₇)₄, Ti(OC₄H₉)₄ and Ti(OC₄H₉)₂C₁₂; addition products and complexes obtained from titanium alkoxide and glycols such as ethylene glycol, or acids such as acetic acid and lactic acid, or alkanolamines such as triethanolamine, or β -diketons such as acetylacetone; and chemicals obtained by dissolving chlorides such as TiC₁₄ in alcohol for general application, such as ethanol, or in solvents such as acetic ester and β -diketone. In view of high activity and transmittance, β -diketone complex solution obtained by dissolving titanium alkoxide in acetylacetone is particularly preferable.

Further, various known methods for accelerating a photocatalytic function can be employed appropriately. For example, a trace quantity of metal (for example, gold, platinum, palladium, silver, copper and so forth) may be carried by the titanium dioxide film.

Moreover, ITO film may be formed on the precoat layer so as to impart an electromagnetic-wave shielding function thereto and thus impart conductiveness thereto. Furthermore, a titanium dioxide film may be formed thereon.

As stated in detail in the foregoing description of the embodiments, characteristic aspects of the titanium dioxide photocatalyst structure of the present invention and the methods for producing the same according to the present invention reside in that a titanium dioxide film which has at least photocatalytic activity and simultaneously has light transmittance corresponding to light having a wavelength of 550 nm is not less than 50 % and is formed on a transparent substrate. Thereby, excellent photocatalytic action and optical transmissivity can be obtained. Moreover, members composing various structures such as a glass window, which are especially required to have optical transmissivity, can further have photocatalytic activities. This respect is very important from the view of substantial enhancement of the light irradiation efficiency and the transparency.

### Industrial Applicability

As above described, the titanium dioxide photocatalyst structure can be used as a member of various structures especially required to have the transparency, for example, a glass window. The present invention can have distinguished advantages in that actions of eliminating carbon dioxide and air pollutants (for example, NOₓ and SOₓ) from indoor space, of deodorizing the indoor space and of making the indoor space antibacterial, soil-resistant and mildew-proof are achieved by the window pane itself without using special equipment. Additionally, the present invention can obtain eminent merits in that in the case of cleaning the room by applying the photocatalyst structure to the window pane, sunlight can be extremely utilized. Moreover, especially, in the case of applying the photocatalyst structure of the present invention to a building or the like, in which glass materials are highly used, of the type that has become common in recent years, the photocatalyst structure of the present invention has immeasurable advantages in cleaning the living space. In addition, the photocatalyst structure of the present invention can be applied to a glass door or the like of a shelf, which includes the door or the like, for storing, for instance, precision devices such as a camera which should be kept away from molds and corrosion. Furthermore, the photocatalyst structure of the present invention can be used for various purposes which require transparent glass. For example, the photocatalyst structure of the present invention can be applied to glass covers for various electronic equipment and instrument, light fixtures such as a fluorescent lamp and a light bulb, a lens and a glass. Thus, the range of application of the photocatalyst structure of the present invention is extremely wide.

## Claims

1. A titanium dioxide photocatalyst structure comprising:
a transparent substrate,
a titanium dioxide film having photocatalytic activity and a light transmittance of at least 50% for light having a wavelength of 550 nm and
a precoat film having transparency and being disposed between the transparent substrate and the titanium dioxide film.

2. The titanium dioxide photocatalyst structure according to claim 1, wherein the titanium dioxide film contains at least an anatase crystal.

3. The titanium dioxide photocatalyst structure according to claim 1 or 2, wherein the precoat film has a thickness of 0.02 to 0.2 µm.

4. The titanium dioxide photocatalyst structure according to any of claims 1 to 3, wherein the transparent substrate is made of glass.

5. The titanium dioxide photocatalyst structure according to any of claims 1 to 4, wherein the precoat film is composed of SiO₂.

6. A method for producing a titanium dioxide photocatalyst structure according to any of claims 1 to 5, comprising a producing process which includes the step of:
forming a titanium dioxide film on a transparent substrate by performing a pyro-sol method, a dipping method, a printing method or a CVD method.

7. Use of the titanium dioxide photocatalyst structure according to any of claims 1 to 5 for removing carbon dioxide and air pollutants to render indoor space antibacterial.
